Europäisches Patentamt

European Patent Office

Office européen des brevets

(19)

(11) Veröffentlichungsnummer: **0 377 177 B1**

## (12) EUROPÄISCHE PATENTSCHRIFT

(45) Veröffentlichungstag der Patentschrift: **02.06.93**

(51) Int. Cl.⁵: **C07D 229/00**, C08G 18/79

(21) Anmeldenummer: **89123684.6**

(22) Anmeldetag: **21.12.89**

(54) **Verfahren zur Herstellung von Uretdion-und Isocyanuratgruppen aufweisenden Polyisocyanaten, die nach diesem Verfahren erhältlichen Polyisocyanate und ihre Verwendung in Zweikomponenten Polyurethanlacken.**

(30) Priorität: **03.01.89 DE 3900053**

(43) Veröffentlichungstag der Anmeldung:
**11.07.90 Patentblatt 90/28**

(45) Bekanntmachung des Hinweises auf die
Patenterteilung:
**02.06.93 Patentblatt 93/22**

(84) Benannte Vertragsstaaten:
**AT BE CH DE ES FR GB IT LI NL SE**

(56) Entgegenhaltungen:
**EP-A- 0 071 899
EP-A- 0 099 976
DE-A- 1 934 763
DE-A- 2 349 726
GB-A- 1 134 285**

(73) Patentinhaber: **BAYER AG**

**W-5090 Leverkusen 1 Bayerwerk(DE)**

(72) Erfinder: **Dell, Winfried, Dr.
Bruchhauser Strasse 99
W-5090 Leverkusen(DE)**
Erfinder: **Kubitza, Werner, Dipl.-Ing.
Eduard-Spranger-Strasse 22
W-5090 Leverkusen(DE)**
Erfinder: **Liebsch, Dietrich, Dr.
Nietzsche Strasse 8
W-5090 Leverkusen(DE)**

**Beschreibung**

Die Erfindung betrifft ein verbessertes Verfahren zur Herstellung von Uretdion- und Isocyanuratgruppen aufweisenden (cyclo)aliphatischen Polyisocyanaten durch entsprechende Modifizierung von einfachen (cyclo)aliphatischen Diisocyanaten unter Verwendung von tertiären Phosphinen als Katalysatoren für die Modifizierungsreaktion, die nach diesem Verfahren erhältlichen modifizierten Polyisocyanate, die sich insbesondere durch besonders niedrige Farbzahlen auszeichnen, sowie deren Verwendung als Isocyanatkomponente in Zweikomponenten-Polyurethanlacken.

Die Herstellung von Uretdion- und Isocyanuratgruppen aufweisenden Lackpolyisocyanaten durch entsprechende Oligomerisierung von einfachen Ausgangsdiisocyanaten unter Verwendung von organischen Phosphinen als Katalysatoren und Abbruch der Modifizierungsreaktion beim jeweils gewünschten Oligomerisierungsgrad ist bekannt (vgl. z.B. DE-OS 1 670 667, DE-OS 1 670 720, DE-OS 1 954 093 oder US-PS 4 614 785).

Bei den Verfahren dieser Vorveröffentlichungen entstehen im allgemeinen Uretdion- und Isocyanuratgruppen aufweisende Polyisocyanate, die eine Iodfarbzahl von mindestens 5 aufweisen, was die Verwendbarkeit der Polyisocyanate in Polyurethanlacken einer farbhellen Einstellung beschränkt,

Es war daher die der Erfindung zugrundeliegende Aufgabe, das im Prinzip aus den genannten Vorveröffentlichungen bekannte Verfahren zur Herstellung von Uretdion- und Isocyanuratgruppen aufweisenden Polyisocyanaten auf Basis von (cyclo)aliphatischen Ausgangsdiisocyanaten so zu verbessern, daß Verfahrensprodukte resultieren, die Iodfarbzahlen von maximal 3, vorzugsweise von 0 bis 1 bzw. Hazen-Farbzahlen gemäß DIN 53 409 von maximal 250, vorzugsweise von unter 100 resultieren.

Diese Aufgabe konnte mit dem nachstehend näher beschriebenen erfindungsgemäßen Verfahren gelöst werden.

Gegenstand der Erfindung ist ein Verfahren zur Herstellung von Uretdion- und Isocyanuratgruppen aufweisenden, modifizierten Polyisocyanaten durch Oligomerisierung eines Teils der Isocyanatgruppen von organischen Diisocyanaten mit (cyclo)aliphatisch gebundenen Isocyanatgruppen in Gegenwart von organischen Phosphinen als Katalysator und Abbruch der Oligomerisierungsreaktion beim gewünschten Oligomerisierungsgrad durch Zugabe eines Katalysatorengifts und anschließende destillative Entfernung der Hauptmenge des überschüssigen, nicht umgesetzten Ausgangsdiisocyanats, sowie nachfolgender Weiterbehandlung des hierbei anfallenden Destillationsrückstands, dadurch gekennzeichnet, daß man

a) vor und/oder während der Durchführung der Oligomerisierungsreaktion 0,1 bis 10 % der im Ausgangsdiisocyanat vorliegenden Isocyanatgruppen durch Zugabe einer entsprechenden Menge mindestens eines Alkohols in Urethangruppen überführt
und

b) den nach der destillativen Entfernung der Hauptmenge des überschüssigen Ausgangsdiisocyanats vorliegenden Destillationsrückstand in Abmischung mit 100 bis 10 000 ppm (Gewicht) eines Peroxids auf Temperaturen von mindestens 50 °C erhitzt.

Gegenstand der Erfindung sind auch die nach diesem Verfahren erhältlichen Uretdion- und Isocyanuratgruppen aufweisenden Polyisocyanate.

Gegenstand der Erfindung ist schließlich auch die Verwendung der nach dem erfindungsgemäßen Verfahren erhältlichen modifizierten Polyisocyanate, gegebenenfalls in mit Blockierungsmitteln für Isocyanatgruppen blockierter Form, als Isocyanatkomponente in Zweikomponenten-Polyurethanlacken.

Ausgangsmaterialien für das erfindungsgemäße Verfahren sind einfache (cyclo)aliphatische Diisocyanate eines unter 300 liegenden Molekulargewichts wie beispielsweise 1,4-Diisocyanatobutan, 1,6-Diisocyanatohexan (HDI), 1,12-Diisocyanatododecan, 1-Isocyanato-3,3,5-trimethyl-5-isocyanatomethyl-cyclohexan (IPDI), 4,4'-Diisocyanatodicyclohexylmethan oder beliebige Gemische derartiger (cyclo)aliphatischer Diisocyanate. Besonders bevorzugt wird HDI als Ausgangsdiisocyanat verwendet.

Für die partielle Urethanisierung der Ausgangsdiisocyanate eignen sich beliebige organische Verbindungen, die eine alkoholische Hydroxylgruppe aufweisen und ansonsten unter den Bedingungen des erfindungsgemäßen Verfahrens inert sind. Vorzugsweise werden für diese Modifizierung jedoch niedermolekulare, ein- oder mehrwertige Alkohole, d.h. insbesondere solche des Molekulargewichtsbereichs 32 bis 200 bzw. beliebige Gemische derartiger Alkohole verwendet. Geeignet sind beispielsweise Methanol, Ethanol, n-Propanol, Isopropanol, n-Butanol, n-Hexanol, 2-Ethyl-1-hexanol, Ethylenglykol, Propylenglykol, die isomeren Butandiole, Hexandiole oder Octandiole, Diethylenglykol, Dipropylenglykol, Glycerin, Trimethylolpropan oder beliebige Gemische derartiger Alkohole.

Als Katalysatoren sind beliebige tertiäre Phosphine geeignet, beispielsweise die in US-PS 4 614 785, Kolonne 4, Zeilen 11 bis 47 beispielhaft genannten Verbindungen. Tri-n-butyl-phosphin ist besonders gut als Modifizierungskatalysator geeignet.

Geeignete Katalysatorengifte sind ebenfalls die für diesen Zweck bislang eingesetzten Verbindungen wie beispielsweise Schwefel, Alkylierungsmittel wie Dimethylsulfat, p-Toluolsulfonsäuremethylester oder Sulfonylisocyanate der in US-PS 4 614 785, Kolonne 5, Zeile 27 bis Kolonne 6, Zeile 35 beispielhaft genannten Art.

Als in der letzten Stufe des erfindungsgemäßen Verfahrens einzusetzende Peroxide eignen sich sowohl anorganische Peroxide wie beispielsweise Wasserstoffperoxid als auch organische Peroxide wie beispielsweise Butanonperoxid, Dicumylperoxid, Dilauroylperoxid, tert.-Butylhydroperoxid, Cumolhydroperoxid oder Dibenzoylperoxid. Butanonperoxid ist bevorzugt.

Zur Durchführung des erfindungsgemäßen Verfahrens, das vorzugsweise mindestens bis zur Beendigung der Oligomerisierungsreaktion unter Inertgas (z.B. Stickstoff) durchgeführt wird, werden zunächst 0,1 bis 10, vorzugsweise 0,5 bis 5 % der im Ausgangsdiisocyanat vorliegenden Isocyanatgruppen durch Zusatz eines oder mehrerer Alkohole der beispielhaft genannten Art in Urethangruppen überführt. Hierzu kann beispielsweise so vorgegangen werden, daß eine entsprechende Menge der Alkoholkomponente bei Raumtemperatur dem vorgelegten Diisocyanat zudosiert wird, worauf sich eine Temperaturerhöhung, beispielsweise auf Temperaturen bis 100° C zwecks Beschleunigung der Urethanisierungsreaktion anschließen kann.

Gleichzeitig oder im Anschluß hieran erfolgt die Oligomerisierung des anurethanisierten Ausgangsdiisocyanats unter Zugabe des Katalysators. Die als Katalysatoren eingesetzten tert. Phosphine werden im allgemeinen in einer Menge von 0,1 bis 5,0 Gew.-%, vorzugsweise 0,2 bis 2,0 Gew.-%, bezogen auf das Gewicht des unmodifizierten Ausgangsdiisocyanats, verwendet. Die Modifizierungsreaktion erfolgt im allgemeinen innerhalb des Temperaturbereichs von 20 bis 100, vorzugsweise 50 bis 80° C. Es ist im übrigen nicht unbedingt erforderlich, daß die Urethanisierungsreaktion beeits völlig beendet ist, bevor der Katalysator zugefügt wird. Beide Reaktionen können auch, zumindest teilweise, gleichzeitig ablaufen.

Nach Erreichen des gewünschten Oligomerisierungsgrad (Oligomerisierungsgrad = Prozentsatz der Isocyanatgruppen des urethanmodifizierten Ausgangsdiisocyanats, die unter Dimerisierung oder Trimerisierung abreagieren) wird die Umsetzung durch Zugabe des Katalysatorgifts abgebrochen. Im allgemeinen erfolgt dieser Abbruch der Reaktion bei einem Oligomerisierungsgrad von 10 bis 80, vorzugsweise 20 bis 30 %. Die Menge des hierzu erforderlichen Katalysatorgifts richtet sich nach der Menge des eingesetzten Trimerisierungskatalysators, wobei jedoch Katalysatorverluste während der Umsetzung berücksichtigt werden können, was zur Folge hat, daß im allgemeinen ca. 20 bis 80 Äquivalent-% Katalysatorgift, bezogen auf den zu Beginn der Reaktion eingesetzten Katalysator ausreichend sind.

Nach beendeter Umsetzung wird die Hauptmenge des nicht umgesetzten, überschüssigen Ausgangsdiisocyanats destillativ in an sich bekannter Weise entfernt und vorzugsweise für einen weiteren Reaktionsansatz wiederverwendet. Diese Destillation kann beispielsweise unter Verwendung der hierfür üblicherweise eingesetzten Fallrohrverdampfer oder Dünnschichtverdampfer erfolgen. Im allgemeinen weisen die hierbei anfallenden Destillationsrückstände einen Restgehalt an monomerem Ausgangsdiisocyanat von weniger als 2 Gew.-%, vorzugsweise von weniger als 0,5 Gew.-% auf.

Gemäß der letzten Stufe des erfindungsgemäßen Verfahrens werden die so erhaltenen Destillationsrückstände in Gegenwart eines der oben beispielhaft genannten Peroxide einer Wärmebehandlung unterzogen. Hierzu werden die Destillationsrückstände mit mindestens einem der beispielhaft genannten Peroxide vermischt, wobei insgesamt 100 bis 10 000, vorzugsweise 500 bis 2000 ppm (Gewicht), bezogen auf das Gewicht des Destillationsrückstands, der Peroxide zum Einsatz gelangen. Das Gemisch aus Destillationsrückstand und Peroxid wird anschließend während eines Zeitraums von mindestens 20 Minuten, vorzugsweise von 30 bis 90 Minuten auf Temperaturen von mindestens 50° C, vorzugsweise innerhalb des Temperaturbereichs von 50 bis 120° C erhitzt.

Die hierbei als Verfahrensprodukte anfallenden, modifizierten Polyisocyanate weisen im Falle der bevorzugten Verwendung von 1,6-Diisocyanatohexan als Ausgangsdiisocyanat einen Isocyanatgehalt von 20 bis 24 Gew.-%, einen Urethangruppengehalt (berechnet als -NH-CO-O-) von 1 bis 5 Gew.-%, eine Iodfarbzahl gemäß DIN 6162 von 0 bis 3 und eine HAZEN-Farbzahl gemäß DIN 53 409 von unter 250 auf. Das Molverhältnis von Uretdiongruppen zu Isocyanuratgruppen liegt im allgemeinen bei 1:1 bis 4:1. Die Viskosität dieser Produkte bei 23° C liegt im allgemeinen bei 100 bis 300 mPa.s.

Die erfindungsgemäßen Verfahrensprodukte insbesondere die bevorzugten erfindungvsgemäßen Verfahrensprodukte auf Basis von 1,6-Diisocyanatohexan stellen besonders wertvolle Lackpolyisocyanate dar. Sie können, gegebenenfalls in mit Blockierungsmitteln für Isocyanatgruppen blockierter Form, in an sich bekannter Weise mit organischen Polyhydroxylverbindungen, insbesondere organischen Polyhydroxypolyestern oder Polyhydroxypolyacrylaten zu hochwertigen Zweikomponenten-Bindemitteln vereinigt werden.

In den nachfolgenden Beispielen beziehen sich alle Prozentangaben auf Gewichtsprozente.

EP 0 377 177 B1

Beispiel 1

a) Urethanisierung mit anschließender Oligomerisierung

In einem geeigneten Reaktionsgefäß werden unter trockenem Stickstoff 1344 g (4 Mol) 1,6-Diisocyanatohexan vorgelegt und bei Raumtemperatur mit 13,4 g (0,092 Mol) 2,2,4-Trimethylpentandiol-1,3 verrührt.

Das Gemisch wird anschließend auf 60° C erhitzt und bei dieser Temperatur belassen, bis die Umsetzung zwischen Isocyanatgruppen und Hydroxylgruppen abgeschlossen ist. Anschließend werden 4,0 g (0,02 Mol) Tri-n-butylphosphin hinzugegeben. Durch externe Kühlung wird die Temperatur während der exothermen Reaktion auf 60° C gehalten. Nach einer Rührzeit von 6 h bei 60° C ist der NCO-Gehalt auf ca. 39 % abgefallen. Die Reaktion wird durch Zugabe von 2,8 g (0,015 Mol) Toluolsulfonsäuremethylester und einer zweistündigen thermischen Nachbehandlung bei 80° C abgebrochen. Nicht umgesetztes Ausgangsdiisocyanat wird anschließend im Dünnschichtverdampfer bei 150° C und einem Druck von unter 0,5 mbar bis auf einen Restgehalt von unter 0,5 % abdestilliert. Das aus Sumpfprodukt anfallende monomerenarme Umsetzungsprodukt weist eine Iodfarbzahl von 1, einen NCO-Gehalt von 21,6 % und eine Viskosität von 150 mPa.s (23° C) auf.

Das anfallende, nicht umgesetzte Ausgangsdiisocyanat wird anschließend erneut zusammen mit frischem 1,6-Diisocyanatohexan zur Durchführung der beschriebenen Reaktion eingesetzt. Dieser Vorgang wird insgesamt 20 mal wiederholt (20 Reaktionszyklen). Am Ende des 20. Reaktionszyklus resultiert als Destillationsrückstand ein monomerenarmes Verfahrensprodukt mit einer Iodfarbzahl von 3. Dieses Produkt wird anschließend erfindungsgemäß weiterverarbeitet. Damit wird bewiesen, daß selbst von einer ungünstigen Iodfarbzahl ausgehend letztendlich Verfahrensprodukte erhalten werden, die eine besonders niedrige HAZEN-Farbzahl aufweisen.

b) Wärmebehandlung in Anwesenheit eines Peroxids

In einem geeigneten Reaktionsgefäß werden in 4 Parallelversuchen jeweils 80 Gew.-Teile des in Beispiel 1a) genannten Destillationsrückstands der Iodfarbzahl 3 mit 250, 500, 1000 bzw. 2500 ppm (Gewicht) Butanonperoxid vermischt (das Butanonperoxid wurde in Form einer 50-gew.-%igen Lösung in Dimethylphthalat verwendet, die Mengenangaben beziehen sich auf die 100 %ige Substanz). Die so erhaltenen Proben werden anschließend jeweils während 1 h auf 100° C erhitzt. Die Abhängigkeit der Farbzahlen von der Menge an Peroxid geht aus nachstehender Tabelle 1 hervor.

Tabelle 1

| ppm Peroxid | Iod-Farbzahl | HAZEN-Farbzahl |
|---|---|---|
| - | 3 | - |
| 250 | 2 | - |
| 500 | 1-2 | - |
| 1000 | 1 | 150 |
| 2500 | <1 | 125 |

Eine Verlängerung der beschriebenen Hitzebehandlung auf insgesamt 4 h führte zu einer weiteren Aufhellung der Produkte. Dies geht aus nachfolgender Tabelle 2 hervor.

4

Tabelle 2

| Zeit (h) | Temp. (°C) | Peroxid (ppm) | | | | | | | |
|---|---|---|---|---|---|---|---|---|---|
| | | 250 | | 500 | | 1000 | | 2500 | |
| | | Iod-FZ | HAZEN | Iod-FZ | HAZEN | Iod-FZ | HAZEN | Iod-FZ | HAZEN |
| 0 | RT | 3 | - | | | | | | |
| 1 | 100 | 2 | - | 1-2 | | 1 | 150 | <1 | 125 |
| 2 | 100 | 1 | 150 | <1 | 125 | <1 | 125 | <1 | 100 |
| 4 | 100 | <1 | 125 | <1 | 125 | <1 | 100 | <1 | 60 |

In einer weiteren Versuchsreihe wurde unter Beibehaltung der Peroxid-Menge (500 ppm Butanonperoxid) die Temperatur und die Dauer der Hitzebehandlung variiert. Die hierbei resultierenden Ergebnisse sind in Tabelle 3 zusammengefaßt.

5

Tabelle 3

| Zeit (h) | 50° C | | 75° C | | 100° C | |
|---|---|---|---|---|---|---|
| | Iod-FZ | HAZEN | Iod-FZ | HAZEN | Iod-FZ | HAZEN |
| 1 | 2 | - | 2 | - | 1-2 | |
| 2 | 1-2 | - | 1-2 | - | <1 | 125 |
| 4 | 1 | 150 | 1-2 | 150 | <1 | 125 |

In einer weiteren Versuchsreihe wurden die Peroxide variiert. Zum Einsatz gelangten Butanonperoxid (50 %ige Lösung in Dimethylphthalat), Dicumylperoxid (50 %ige Lösung in Ethylacetat), Dilauroylperoxid (30 %ige Lösung in Methylenchlorid), tert.-Butylhydroperoxid (50 %ige Lösung in Ethylacetat), Cumolhydroperoxid (50 %ige Lösung in Ethylacetat), Dibenzoylperoxid (20 %ige Lösung in Methylenchlorid) und Wasserstoffperoxid (30 %ige Lösung in Wasser). Die Peroxide gelangten jeweils in einer solchen Menge zum Einsatz, die einer Konzentration von 75 ppm an aktivem Sauerstoff (O) entspricht. Die Menge der zum Einsatz gelangenden Peroxide, bezogen auf deren Gesamtgewicht, lag somit stets innerhalb des Bereichs von 100 bis 10 000 ppm. Die Ergebnisse sind in nachstehender Tabelle 4 zusammengefaßt.

Tabelle 4

| Zeit (h) | Temp. (°C) | Peroxid | Iod-FZ |
|---|---|---|---|
| 0 | RT | - | 3 |
| 2 | 100 | - | 3-4 |
| 2 | 100 | Butanonperoxid | 1-2 |
| 2 | 100 | Dicumylperoxid | 2 |
| 2 | 100 | Dilauroylperoxid | 2 |
| 2 | 100 | tert.-Butylperoxid | 2 |
| 2 | 100 | Cumolhydroperoxid | 2 |
| 2 | 100 | Dibenzoylperoxid | 2 |
| 2 | 100 | Wasserstoffperoxid | 2 |

Bei einer Variation der zum Einsatz gelangenden Alkoholkomponente, beispielsweise unter Verwendung äquivalenter Mengen an Diethylenglykol, Butandiol-1,3, 2-Ethylhexandiol-1,3, 2-Ethyl-hexanol-1, Isopropanol oder Methanol werden weitgehend entsprechende Resultate erzielt.

Beispiel 2

a) Urethanisierung und Oligomerisierung

2000 kg 1,6-Diisocyanatohexan werden in einem geeigneten Reaktionsgefäß vorgelegt und auf 50° C erhitzt. Unter ständigem Rühren unter Stickstoffatmosphäre werden anschließend 20 kg 2,2,4-Trimethylpentandiol-1,3 und dann 30 kg Tri-n-butylphosphin eingetragen. Die exotherme Reaktion wird durch Kühlung auf 60° C gehalten. Nach einer Reaktionszeit von 6 h weist das Reaktionsgemisch einen NCO-Gehalt von 42,5 % auf. Die Reaktion wird jetzt durch Zugabe von 16,5 kg Toluolsulfonsäuremethylester und 2 stündiges Nacherhitzen bei 80° C abgestoppt. Die so erhaltene Rohware wird dann mittels Fallrohrverdampfer (165° C/1 mbar) und Dünnschichtverdampfer (150° C/0,30 mbar) von überschüssigem Ausgangsdiisocyanat befreit.

Das resultierende Produkt weist folgende Daten auf:
NCO-Gehalt (%) 21,6
Viskosität (mPa.s/23° C) <200
HAZEN-Farbzahl <100
freies Ausgangsdiisocyanat (%) 0,5

b) Thermische Nachbehandlung in Gegenwart eines Peroxids

Die gemäß Beispiel 2a) erhaltene Rohware wird mit 500 ppm (Gewicht) Butanonperoxid vermischt (zum Einsatz gelangte eine 50 %ige Lösung in Dimethylphthalat, die Mengenangabe bezieht sich auf 100 %igen Wirkstoff). Das so erhaltene Gemisch wird anschließend während 1 h auf 80° C erhitzt. Es resultierte eine HAZEN-Farbzahl von unter 40.

**Patentansprüche**

1. Verfahren zur Herstellung von Uretdion- und Isocyanuratgruppen aufweisenden, modifizierten Polyisocyanaten durch Oligomerisierung eines Teils der Isocyanatgruppen von organischen Diisocyanaten mit (cyclo)aliphatisch gebundenen Isocyanatgruppen in Gegenwart von organischen Phosphinen als Katalysator und Abbruch der Oligomerisierungsreaktion beim gewünschten Oligomerisierungsgrad durch Zugabe eines Katalysatorengifts und anschließende destillative Entfernung der Hauptmenge des überschüssigen, nicht umgesetzten Ausgangsdiisocyanats, sowie nachfolgender Weiterbehandlung des hierbei anfallenden Destillationsrückstands, dadurch gekennzeichnet, daß man
   a) vor und/oder während der Durchführung der Oligomerisierungsreaktion 0,1 bis 10 % der im Ausgangsdiisocyanat vorliegenden Isocyanatgruppen durch Zugabe einer entsprechenden Menge mindestens eines Alkohols in Urethangruppen überführt
   und
   b) den nach der destillativen Entfernung der Hauptmenge des überschüssigen Ausgangsdiisocyanats vorliegenden Destillationsrückstand in Abmischung mit 100 bis 10 000 ppm (Gewicht) eines Peroxids auf Temperaturen von mindestens 50° C erhitzt.

2. Verfahren gemäß Anspruch 1, dadurch gekennzeichnet, daß man als Ausgangsdiisocyanat 1,6-Diisocyanatohexan verwendet.

3. Verfahren gemäß Anspruch 1 und 2, dadurch gekennzeichnet, daß man als Alkohole ein- oder mehrwertige aliphatische Alkohole des Molekulargewichtsbereichs 32 bis 200 oder Abmischungen mehrerer derartiger Alkohole verwendet.

4. Verfahren gemäß Anspruch 1 bis 3, dadurch gekennzeichnet, daß man als Peroxid Butanonperoxid verwendet.

5. Gemäß Anspruch 1 bis 4 erhältliche, modifizierte Polyisocyanate.

**6.** Verwendung der gemäß Anspruch 1 bis 4 erhältlichen, modifizierten Polyisocyanate, gegebenenfalls in mit Blockierungsmitteln für Isocyanatgruppen blockierter Form als Isocyanatkomponente in Zweikomponenten-Polyurethanlacken.

## Claims

**1.** A process for the production of modified polyisocyanates containing uretdione and isocyanurate groups by partial oligomerization of the isocyanate groups of organic diisocyanates containing (cycloaliphatically) bound isocyanate groups in the presence of organic phosphines as catalysts and termination of the oligomerization reaction at the desired degree of oligomerization by addition of a catalyst poison, subsequent removal of most of the excess unreacted starting diisocyanate by distillation and further treatment of the distillation residue accumulating, characterized in that

a) before and/or during the oligomerization reaction, 0.1 to 10% of the isocyanate groups present in the starting diisocyanate are converted into urethane groups by addition of a corresponding quantity of at least one alcohol

and

b) the distillation residue present after removal of most of the excess starting diisocyanate by distillation is heated to temperatures of at least 50°C in admixture with 100 to 10,000 ppm (weight) of a peroxide.

**2.** A process as claimed in claim 1, characterized in that 1,6-diisocyanatohexane is used as the starting diisocyanate.

**3.** A process as claimed in claims 1 and 2, characterized in that mono- or polyhydric aliphatic alcohols having a molecular weight in the range from 32 to 200 or mixtures of several such alcohols are used as the alcohols.

**4.** A process as claimed in claims 1 to 3, characterized in that butanone peroxide is used as the peroxide.

**5.** Modified polyisocyanates obtainable by the process claimed in claims 1 to 4.

**6.** The use of the modified polisocyanates obtainable by the process claimed in claims 1 to 4, optionally blocked with blocking agents for isocyanate groups, as isocyanate component in two-component polyurethane lacquers.

## Revendications

**1.** Procédé de préparation de polyisocyanates modifiés à groupes uretdiones et isocyanurates par oligomérisation d'une partie des groupes isocyanates de diisocyanates organiques avec des groupes isocyanates liés (cyclo)aliphatiquement en présence de phosphines organiques comme catalyseur, par interruption de la réaction d'oligomérisation quand le degré d'oligomérisation souhaité est atteint par addition d'un poison du catalyseur, puis séparation par distillation de la majeure partie du diisocyanate de départ n'ayant pas réagi, en excès, et traitement ultérieur suivant du résidu de distillation ainsi obtenu, caractérisé en ce qu'on:

a) transforme 0,1 à 10 % des groupes isocyanates présents dans le diisocyanate de départ en groupes uréthannes par addition d'une quantité correspondante d'au moins un alcool avant et/ou pendant la mise en oeuvre de la réaction d'oligomérisation,

et

b) chauffe le résidu de distillation présent après la séparation par distillation de la majeure partie du diisocyanate de départ en excès mélangé avec 100 à 10 000 ppm (en poids) d'un peroxyde à une température d'au moins 50°C.

**2.** Procédé selon la revendication 1, caractérisé en ce qu'on utilise comme diisocyanate de départ le 1,6-diisocyanatohexane.

**3.** Procédé selon les revendications 1 et 2, caractérisé en ce qu'on utilise comme alcools des alcools aliphatiques mono- ou plurivalents dont le poids moléculaire se situe dans un domaine allant de 32 à 200 ou des mélanges de plusieurs alcools de cette nature.

4. Procédé selon les revendications 1 à 3, caractérisé en ce qu'on utilise comme peroxyde le peroxyde de butanone.

5. Polyisocyanates modifiés que l'on peut obtenir selon les revendications 1 à 4.

6. Utilisation des polyisocyanates modifiés que l'on peut obtenir selon les revendications 1 à 4, le cas échéant sous forme bloquée à l'aide d'agents de blocage pour les groupes isocyanates, comme composant isocyanate dans des laques de polyuréthanne à deux composants.